# EUROPEAN PATENT APPLICATION

(11) **EP 4 617 657 A1**
(43) Date of publication of application: **17.09.2025**
(21) Application number: 24219086.6
(22) Date of filing: 11.12.2024
(51) Int. Cl.: G01N 33/00

(54) **ODOR DETECTION SYSTEM**

(30) Priority: 11.03.2024 KR 20240033726; 27.03.2024 KR 20240041894
(71) Applicant: Hyundai Motor Company, Seoul 137-938 (KR); Kia Corporation, Seoul (KR)
(72) Inventor: LEE, Taehee, 18280 Hwaseong-si, Gyeonggi-do, (KR); SUNG, Dae Un, 18280 Hwaseong-si, Gyeonggi-do, (KR)
(74) Representative: Viering, Jentschura & Partner mbB Patent- und Rechtsanwälte

(57) **Abstract**

An embodiment odor detection system includes a chamber assembly including a chamber, a controller configured to control a temperature and a humidity of a target gas and inflow the target gas into the chamber, and a sensor configured to detect an odor of the target gas within the chamber.

## Description

### CROSS-REFERENCE TO RELATED APPLICATIONS

This application claims the benefit of Korean Patent Application No. 10-2024-0033726, filed on March 11, 2024, and Korean Patent Application No. 10-2024-0041894, filed on March 27, 2024, which applications are hereby incorporated herein by reference.

### TECHNICAL FIELD

The present disclosure relates to a system for detecting odor.

### BACKGROUND

For odor detection devices, electrochemical sensors, bio-peptide type sensors using surface plasmon resonance, or sensors using amino acids have been developed.

The electrochemical sensors have the problem that the accuracy of the sensor itself is low and the durability lifespan is very short. The bio-peptide type sensors or amino acid sensors have the merit of reacting precisely to odor molecules, but they have the drawback of being sensitive to temperature and humidity. For sensors like the ones above, when humidity is high, the reactivity for the odor molecules increases, which can lead to errors such as measuring other gas data as the odor.

### SUMMARY

The present disclosure relates to a system for detecting odor. Particular embodiments relate to a system for detecting odor, a chamber for detecting the odor, and a control method thereof.

Some embodiments provide an odor detection system. The odor detection system includes a chamber assembly including a chamber receiving target gas, a controller configured to control temperature and humidity of the target gas and inflow the controlled target gas into the chamber, and a sensor device configured to detect odor of the controlled target gas within the chamber.

The controller may be further configured to maintain internal temperature of the chamber at a predetermined temperature and maintain internal humidity of the chamber at a predetermined humidity.

The chamber assembly may further include an inlet and an outlet. The controlled target gas may flow into the chamber through the inlet, and gas in the chamber may flow out of the chamber through the outlet.

The system may further include a heating device configured to be coupled to the inlet to increase the temperature of gas in the inlet.

The controller may be further configured to inflow, into the chamber, the target gas of which the temperature and humidity are controlled by the heating device.

The controller may be further configured to control the heating device based on temperature and humidity of the target gas measured on both sides of the heating device.

The controller may be further configured to control a blocking member of the inlet while the temperature and humidity of the target gas in the inlet are controlled by the heating device, and the blocking member may be positioned on one side of the inlet close to the chamber.

The inlet may include a plurality of bent portions, and the heating device may surround the plurality of bent portions.

The heating device may include a coil-shaped heat radiating device, and the inlet may pass through a portion surrounded by the heat radiating device.

The controller may be further configured to control flow through the inlet and the outlet by blocking or opening some or all of the blocking members of the inlet and the outlet.

The controller may be further configured to control a pump device of the inlet and the outlet to regulate speed and flow rate of fluid through the inlet and the outlet.

The system may further include an air tank and a valve, wherein the controller may be further configured to operate the valve to inject preset gas contained in the air tank into the chamber, and temperature and humidity of the preset gas may be adjusted to predetermined values.

The sensor device may be further configured to perform an initialization using the preset gas contained in the chamber.

The system may further include an outside air intake and a valve, wherein the controller may be further configured to operate the valve to inject outside air sucked through the outside air intake into the chamber.

The sensor device may be further configured to perform an initialization using the outside air contained in the chamber.

The sensor device may be further configured to detect the odor from mixed gas of the target gas and remaining gas in the chamber in response to the remaining gas and the target gas being mixed within the chamber.

The sensor device may be further configured to start operations to detect the odor in response to temperature and humidity of the mixed gas reaching the target value.

The sensor device may be further configured to start operations to detect the odor in response to speed or flow rate of the mixed gas becoming stable.

The system may further include a circulation flow path coupled with the inlet and the outlet, wherein the controller may be further configured to control a pump member to circulate the target gas within a closed path formed by the circulation flow path.

The sensor device may be further configured to detect the odor while the target gas is circulating within the closed path or detect the odor after the target gas has circulated within the closed path for a predetermined time.

The controller may be further configured to block blocking members of the inlet and the outlet in response to the odor being detected by the sensor device, and the blocking members may be positioned on one side of the inlet and the outlet close to the chamber.

### BRIEF DESCRIPTION OF THE DRAWINGS

FIG. 1 illustrates an odor detection system according to some embodiments.
FIG. 2A is a perspective view of a chamber assembly according to some embodiments.
FIG. 2B is a top plan view of a chamber assembly according to some embodiments.
FIG. 2C to FIG. 2E are front views of a chamber assembly according to some embodiments.
FIG. 3 illustrates a chamber assembly coupled with an air tank according to some embodiments.
FIG. 4 illustrates a chamber assembly coupled with an outside air intake according to some embodiments.
FIG. 5A to FIG. 5C illustrate a chamber assembly coupled with a heating device according to some embodiments.
FIG. 6 illustrates a circulation flow path and the coupled chamber assembly according to some embodiments.

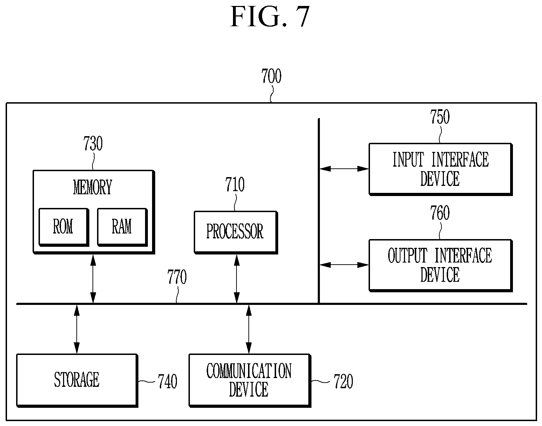

FIG. 7 illustrates the controller of the odor detection system according to another embodiment.

### DETAILED DESCRIPTION OF ILLUSTRATIVE EMBODIMENTS

Hereinafter, with reference to the accompanying drawings, some embodiments of the present disclosure will be described in detail and thus a person of ordinary skill in the art can easily practice them in the technical field to which the present disclosure is included. However, the embodiments of the present disclosure may be implemented in several different forms and are not limited to the embodiments described herein. Accordingly, the drawings and description are to be regarded as illustrative in nature and not restrictive, and like reference numerals designate like elements throughout the specification.

In addition, unless explicitly described to the contrary, the word "comprise," and variations such as "comprises" or "comprising," will be understood to imply the inclusion of stated elements but not the exclusion of any other elements.

In this specification, "A or B," "at least one of A and B," "at least one of A or B," "A, B, or C," "at least one of A, B, and C," and "at least one of A, B, or C" may each include any one of the items listed together in the corresponding phrase or any possible combination thereof.

In this specification, expressions described in the singular may be construed in the singular or plural unless an explicit expression such as "one" or "single" is used.

As used herein, "and/or" includes each and every combination of one or more of the recited elements.

In the specification, it will be understood that, although the terms "first," "second," and the like may be used herein to describe various elements, these elements should not be limited by these terms. These terms are only used to distinguish one element from another. For example, a first element could be termed a second element, and a second element could similarly be termed a first element without departing from the scope of the embodiments of the present invention.

In a flowchart described with reference to drawings in this specification, the order of operations may be changed, several operations may be merged, some operations may be divided, and specific operations may not be performed.

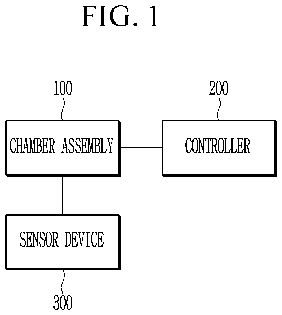

FIG. 1 illustrates an odor detection system according to some embodiments.

Referring to FIG. 1, an odor detection system according to some embodiments may include a chamber assembly 100, a controller 200, and a sensor device 300. The controller 200 of the odor detection system may inflow air into the chamber assembly 100, and the sensor device 300 may detect odor from the air staying in the chamber assembly 100. Before entering the chamber assembly 100, the air may stay in a relatively small, isolated space from the outside, such as the inside of a vehicle.

The chamber assembly 100 may include a chamber that receives a target gas. The target gas may inflow into the chamber and outflow to the outside of the chamber under the control of the controller 200. The target gas may be mixed with air remaining in the chamber, and the sensor device 300 may detect odor from the mixed air of the target gas and the remaining air in the chamber.

The controller 200 may regulate speed and/or flow rate of the air inside the chamber while the sensor device 300 performs the detecting of the odor. The controller 200 may regulate the speed and/or flow rate of the air inside the chamber by controlling a pump device of the inlet/outlet of the chamber assembly 100. The pump device may be positioned adjacent to or near the inlet and/or the outlet. By controlling the speed and flow rate of the air inside the chamber passing through the sensor device 300, the controller 200 may improve accuracy of the performance of the odor detection by the sensor device 300.

Additionally, the controller 200 may maintain the temperature and humidity of the air flowed into the chamber at predetermined target values. The target values of the temperature and/or humidity may be values at which the odor is best detected. The target values of the temperature and/or humidity may be predetermined by the controller 200 or may be input from the sensor device 300.

Additionally, the controller 200 may control environmental variables inside the chamber to maintain an internal temperature of the chamber at a predetermined temperature and/or maintain an internal humidity of the chamber at a predetermined humidity. The controller 200 may control a temperature control device (e.g., heater, cooler, etc.) that controls the temperature of the target gas in the isolated space, such as the inside of the vehicle. The temperature sensor and the humidity sensor may be placed in locations where they are less likely to be affected by other devices.

The sensor device 300 may detect an odor of the target gas contained within the chamber. The sensor device 300 may include at least one sensor module 310 to detect the odor from the target gas. When the temperature and the humidity of the mixed gas inside the chamber are set to the target values by the controller 200, the sensor device 300 may initiate sensing operations to detect the odor. Alternatively, the sensor device 300 may initiate the sensing operations for detecting the odor when the speed and/or flow rate of the mixed gas inside the chamber is stabilized by the controller 200.

The sensor device 300 may obtain real-time sensor data through sensing for the odor detection. The sensor device 300 may check the change in the sensor data derived from the sensor module 310 in the time domain to determine an increase or a decrease in the odor component. The maximum value of the real time sensor data may be used to determine the intensity of the odor.

Additionally, the sensor device 300 may determine the range of the sensor data measured during a predetermined time interval. The range of the sensor data may be used to compare types of odors and classify the odors. At this time, an average value of the sensor data during the predetermined time interval may be used to determine the range of the sensor data.

By accurately controlling the temperature and the humidity of the target gas flowed into the chamber and controlling the speed and flow rate of the gas passing through the chamber, the odor detection system may accurately detect the odor from the target gas in the chamber.

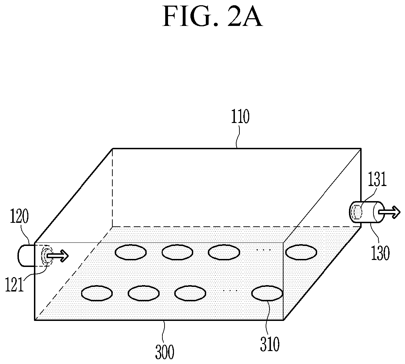

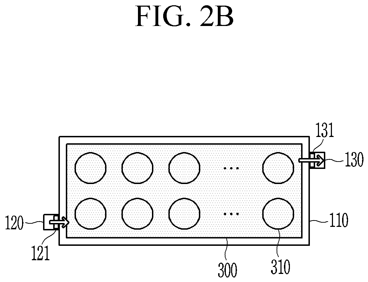

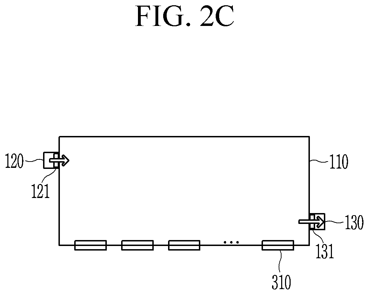

FIG. 2A is a perspective view of a chamber assembly according to some embodiments, FIG. 2B is a top plan view of a chamber assembly according to some embodiments, and FIG. 2C to FIG. 2E are front views of a chamber assembly according to some embodiments.

Referring to FIG. 2A to FIG. 2E, the chamber assembly 100 may include a chamber 110, an inlet 120, and an outlet 130. In FIG. 2A to FIG. 2E, the chamber 110 is shown as a cuboid, but this is simplified to explain the function of the chamber 110, and a shape of the chamber 110 may be modified according to a specific embodiment and is not limited to the depicted figures.

In FIG. 2A to FIG. 2E, arrows may indicate flows of the target gas entering the inside of the chamber 110 through the inlet 120 and flows of air going out of the chamber 110 through the outlet 130.

The chamber 110 may be a space to contain the target gas through which the target gas flows. The air inside, for example, a vehicle where the vehicle's driver, passengers, etc. are positioned may be flowed into the chamber 110 through the inlet 120 by the controller 200 for the odor detection, and when the odor detection by the sensor device 300 is completed, the air may flow out to the outside of the chamber 110 through the outlet 130. During the odor detection by the sensor device 300, the controller 200 may block the inlet 120 and the outlet 130 to prevent air from entering the chamber 110 or escaping from the chamber 110.

The inlet 120 may be a passage through which the target gas enters the inside of the chamber 110 from the outside of the chamber 110. The inlet 120 may have one port or two or more ports. The controller 200 may control the inflow amount and/or inflow speed of the target gas by manipulating the pump device positioned adjacent to or near the inlet 120.

In some embodiments, the controller 200 may control the flow of the target gas flowing into the chamber 110 and the flow of the air flowing out of the chamber 110 by organically manipulating at least one of blocking members and the pump device of the inlet 120 and the outlet 130.

The blocking member of the inlet 120 and the outlet 130 may be positioned on one side of the inlet 120 and the outlet 130 that is in contact with the chamber 110 (e.g., a first blocking member) and may be positioned on the other side that is not in contact with the chamber 110 (e.g., a second blocking member). The controller 200 may block fluid from passing through the inlet 120 and the outlet 130 by adjusting the blocking member to a blocking state. The controller 200 may allow fluid to pass through the inlet 120 and the outlet 130 by adjusting the blocking member to an open state.

The controller 200 may control the flow of fluid through the inlet 120 and the outlet 130 by blocking or opening some or all of the blocking members.

The controller 200 may prevent air in the chamber 110 from escaping by blocking the first blocking member of the outlet 130 and the inlet 120 during the sensing of the sensor device 300.

The pump device may be positioned inside the inlet 120 and the outlet 130 or outside the inlet 120 and the outlet 130, respectively. The pump device positioned outside of the inlet 120 and the outlet 130 may be positioned adjacent to the inlet 120 and the outlet 130 or near the inlet 120 and the outlet 130. The pump devices of the inlet 120 and the outlet 130 may be installed in a replaceable manner. The controller 200 may adjust the speed and/or flow rate of the fluid through the inlet 120 and the outlet 130 by controlling the pump devices of the inlet 120 and the outlet 130. Laminar flows may be induced within the chamber 110 by the pump device of the inlet 120 and the outlet 130.

In some embodiments, the positions of the inlet 120 and the outlet 130 may be determined so that a distance between the inlet 120 and the outlet 130 is maximized, considering the shapes of the inlet 120 and the outlet 130. Referring to FIG. 2A to FIG. 2E, the inlet 120 and the outlet 130 may be positioned near the two furthest vertices of the cuboid, respectively. By positioning the inlet 120 and the outlet 130 to be far apart from each other, the flow path of the target gas may be ensured to be long within the chamber 110 and the odor detection performance of the sensor device 300 may be improved.

In some embodiments, the sensor device 300 may be positioned on the inner surface of the chamber 110. In order for the sensor device 300 to detect the odor from the air in the chamber 110, a part or all of the inner surface of the chamber 110 in contact with the sensor device 300 may be removed by drilling or the like.

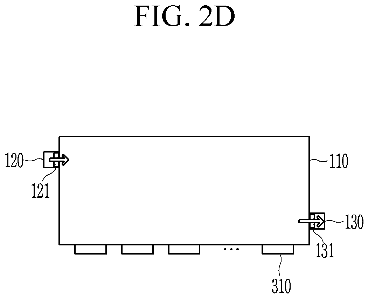

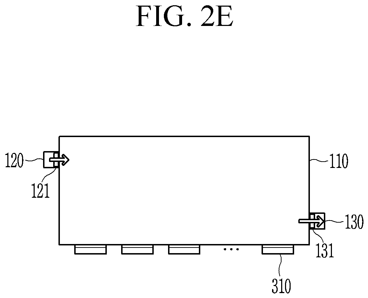

Referring to FIG. 2C to FIG. 2E, the sensor device 300 may be positioned on the bottom of the chamber 110. Referring to FIG. 2C, the sensor device 300 may be arranged so that a portion of the sensor device 300 is protruded on the bottom of the chamber 110. Referring to FIG. 2D, the sensor device 300 may be embedded so that the sensor device 300 is smoothly connected to the bottom of the chamber 110 without any protruding parts on the bottom of the chamber 110. Referring to FIG. 2E, the sensor device 300 may be buried at a predetermined depth so as to be sunken into the bottom of the chamber 110. In some embodiments, the relative positions of the chamber 110 and the sensor device 300 are not limited to what is depicted in the figures and may be modified according to the specific embodiment.

Referring to FIG. 2A to FIG. 2E, temperature and humidity sensors 121 and 131 for monitoring and measuring the temperature and humidity of the target gas may be located at the inlet 120 and outlet 130, respectively. In some embodiments, the controller 200 may control the temperature and humidity of the target gas flowed into the chamber 110 based on the temperature and humidity measured by the temperature and humidity sensor 121 of the inlet 120 and the temperature and humidity sensor 131 of the outlet 130.

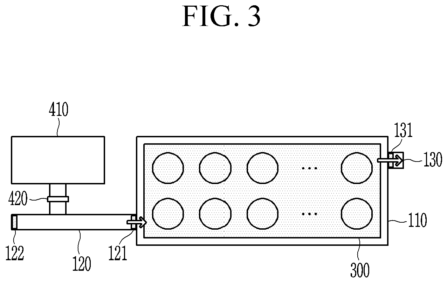

FIG. 3 illustrates a chamber assembly coupled with an air tank according to some embodiments.

The odor detection system according to some embodiments may further include an air tank 410 and a valve 420. Referring to FIG. 3, the air tank 410 and the valve 420 may be coupled to the inlet 120 of the chamber assembly 100.

The air tank 410 may contain clean preset gas that does not include odor molecules. The temperature and humidity of the preset gas contained in the air tank 410 may be adjusted to predetermined values by the controller 200. The controller 200 may control the valve 420 to mix the preset gas in the air tank 410 with the target gas entering the inlet 120, thereby adjusting the temperature and/or humidity of the target gas to the target value.

In some embodiments, the controller 200 may inflow the preset gas in the air tank 410 into the chamber 110 to initialize the sensor device 300. For the initialization of the sensor device 300, the controller 200 may block the target gas from entering through the inlet 120 and allow the gas in the air tank 410 to inflow into the inside of the chamber 110. The controller 200 may execute the operations for the initialization of the sensor device 300 in response to a request from the sensor device 300. The sensor device 300 may perform the initialization using the preset gas included inside the chamber 110.

The preset gas in the air tank 410 may be used to clean a surface of the sensor module 310. The controller 200 may operate the valve 420 to allow the preset gas in the air tank 410 to flow into the inlet 120 and operate the pump device in the inlet 120 to inject the preset gas into the chamber 110 with a strong pressure. The preset gas injected into the chamber 110 may cause the mixed gas in the chamber 110 to be flowed out through the outlet 130, which may have the effect of cleaning the surface of the sensor module 310.

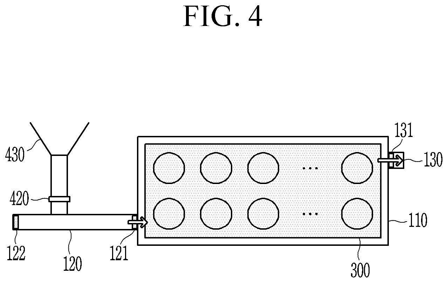

FIG. 4 illustrates a chamber assembly coupled with an outside air intake according to some embodiments.

The odor detection system according to some embodiments may further include an outside air intake 430 and a valve 420. Referring to FIG. 4, the outside air intake 430 and the valve 420 may be coupled to the inlet 120 of the chamber assembly 100. The controller 200 may open the valve 420 and inject outside air sucked through the outside air intake 430 into the chamber 110 through the inlet 120. Before the outside air is injected into the chamber 110, the controller 200 or the sensor device 300 may determine that no odor molecules are substantially present in the outside air or very few are present in the outside air.

The controller 200 may control the temperature and/or humidity of the target gas to the target value by manipulating the valve 420 to mix the outside air with the target gas entering the inlet 120.

The controller 200 may initialize the sensor device 300 using outside air sucked through the outside air intake 430. For the initialization of the sensor device 300, the controller 200 may block the target gas from entering through the inlet 120 and allow the outside air to flow into the inside of the chamber 110. The controller 200 may execute the operations for the initialization of the sensor device 300 in response to a request from the sensor device 300. The sensor device 300 may perform the initialization using the outside air included in the chamber 110.

The outside air may be used to clean a surface of the sensor module 310. The controller 200 may operate the valve 420 to allow the outside air coming through the outside air intake 430 to flow into the inlet 120 and operate the pump device of the inlet 120 to inject the outside air into the chamber 110 with a strong pressure. The outside air injected into the chamber 110 may cause the mixed gas in the chamber 110 to be exhausted through the outlet 130, which may have the effect of cleaning the surface of the sensor module 310.

In FIG. 3 and FIG. 4, temperature and humidity sensors (121, 122, and 131) for monitoring and measurement of the temperature and humidity of the target gas, the preset gas, and/or the outside air may be located at the inlet 120 and the outlet 130.

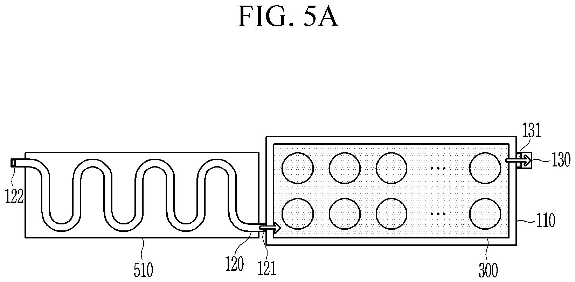

FIG. 5A to FIG. 5C illustrate a chamber assembly coupled with a heating device according to some embodiments.

In some embodiments, the odor detection system may further include a heating device 510, 520. The heating device 510, 520 may be coupled to the inlet 120 of the chamber assembly 100 to increase the temperature of the gas (target gas, outside air, preset gas, mixed gas, etc.) in the inlet 120 and indirectly adjust the relative humidity of the air in the inlet 120.

The controller 200 may control the heating device 510, 520 to adjust the temperature and/or humidity of the target gas in the inlet 120 to a target value. While the temperature and/or humidity of the target gas is controlled, the controller 200 may block the target gas whose temperature and/or humidity is not controlled from the chamber 110 by blocking the first blocking member of the inlet 120. That is, after controlling the temperature and/or humidity of the target gas to the target value, the controller 200 may open the first blocking member of the inlet 120 to inflow the target gas with the temperature and/or humidity of the target value into the chamber 110.

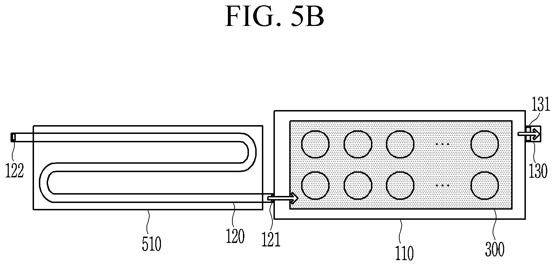

Referring to FIG. 5A and FIG. 5B, the inlet 120 may include a plurality of bent portions, and the heating device 510 may surround the plurality of bent portions. The plurality of bent portions included in the inlet 120 may have a shape curved up and down (column shape) (FIG. 5A) or a shape curved left and right (row shape) (FIG. 5B).

The plurality of bent portions of the inlet 120 may increase the surface area for heat emission from the heating device 510, thereby improving the thermal efficiency of the heating device 510. In FIG. 5A and FIG. 5B, the inlet 120 includes the plurality of bent portions, but the present description is not limited thereto, and the inlet 120 may include various structures that may increase the surface area for the heat emission from the heating device 510.

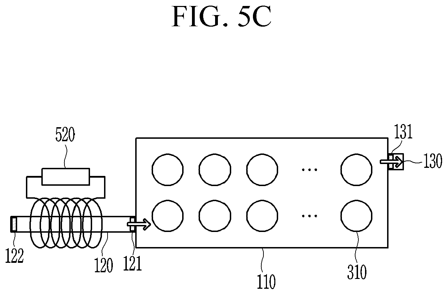

Referring to FIG. 5C, the heating device 520 may include a coil-shaped heat radiating device, and the inlet 120 may pass through the inside of the heat radiating device. That is, the heat radiating device may be in the form of a coil surrounding the inlet 120. The heat emitted from the heat radiating device may be transferred from the heat radiating device to the inlet 120 in the form of radiation and/or may be transferred to the inlet 120 in the form of convection by a medium (gas or liquid) between the heat radiating device and the inlet 120.

In some embodiments, the heating device 510, 520 may collect waste heat generated from electron devices (processors, memory, etc.) of the controller 200 and transfer the heat collected from the electron devices to the inlet 120. Alternatively, the heating device 510, 520 may collect waste heat generated around the odor detection system and transfer the collected heat to the inlet 120.

In FIG. 5A to FIG. 5C, temperature and humidity sensors 121, 122, and 131 for monitoring and measuring the temperature and humidity of the target gas may be located at the inlet 120 and the outlet 130. In some embodiments, a first temperature and humidity sensor 121 of the inlet 120 may be positioned on one side of the heating device 510, 520 and a second temperature and humidity sensor 122 of the inlet 120 may be positioned on the other side of the heating device 510, 520.

In some embodiments, the controller 200 may control the heating device 510, 520 based on the temperature and humidity measured by the temperature and humidity sensors 121 and 122 of the inlet 120 positioned on both sides of the heating device 510, 520 to optimize the temperature and humidity of the target gas injected into the chamber 110. For example, when the temperature of the target gas is measured lower than the optimal temperature by the second temperature and humidity sensor 122 of the inlet 120, the controller 200 may operate the heating device 510, 520 such that the temperature of the target gas is measured by the first temperature and humidity sensor 121 of the inlet 120 to be at the optimal temperature. At this time, the controller 200 may determine operation time and operation intensity of the heating device 510, 520 based on a length of the flow path connected to the inlet 120 and speed of the target gas flowing through the flow path.

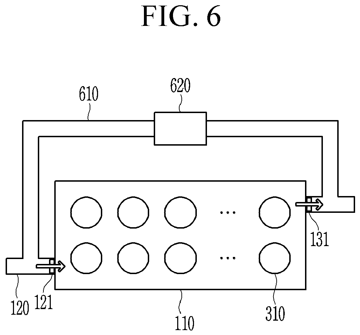

FIG. 6 illustrates a chamber assembly coupled with a circulation flow path according to some embodiments.

Referring to FIG. 6, the odor detection system may include a circulation flow path coupled to the inlet 120 and the outlet 130 of the chamber assembly 100. Air exhausted from the outlet 130 may flow into the inlet 120 through the circulation flow path 610 and may flow back into the chamber 110 through the inlet 120.

In FIG. 6, the temperature and humidity sensors 121 and 131 for monitoring and measuring the temperature and humidity of the target gas may be located at the inlet 120 and the outlet 130.

In some embodiments, the controller 200 may control the pump member 620 of the circulation flow path 610 to return the gas in the chamber 110 coming out through the outlet 130 to the inlet 120. The controller 200 may control the pump member 620 to circulate the target gas in a closed path formed by the circulation flow path 610. The controller 200 may circulate the gas of the chamber 110 within the chamber assembly 100 by manipulating the second blocking member of the inlet 120 and the outlet 130.

When the controller 200 circulates the gas in the chamber 110 within the closed path by the circulation flow path 610, the sensor device 300 may perform the odor detection while the gas in the chamber 110 is circulated or after the gas in the chamber 110 circulates for a predetermined time.

As described above, the controller 200 accurately controls the temperature and humidity of the target gas flowed into the chamber and adjusts the speed and flow rate of the gas passing through the chamber, so that the odor detection system may accurately detect the odor from the target gas in the chamber.

## Claims

1. An odor detection system, the system comprising:
a chamber assembly comprising a chamber;
a controller configured to control a temperature and a humidity of a target gas and inflow the target gas into the chamber; and
a sensor configured to detect an odor of the target gas within the chamber.

2. The system of claim 1, wherein the controller is further configured to maintain a temperature inside the chamber at a predetermined temperature and maintain an internal humidity of the chamber at a predetermined humidity.

3. The system of claim 1 or 2, wherein:
the chamber assembly further comprises an inlet and an outlet; and
the target gas is configured to flow into the chamber through the inlet and flow out of the chamber through the outlet.

4. The system of claim 3, further comprising a heating device configured to be coupled to the inlet to increase the temperature of the target gas flowing in the inlet to control the temperature and a humidity of the target gas, wherein, optionally, the controller is further configured to inflow into the chamber the target gas of which the temperature and the humidity are controlled by the heating device.

5. The system of claim 4,
wherein the controller is further configured to control the heating device based on the temperature and the humidity of the target gas measured on both sides of the heating device, and/or
wherein the controller is further configured to control a blocking member of the inlet while the temperature and the humidity of the target gas in the inlet are controlled by the heating device, and the blocking member is disposed on one side of the inlet close to the chamber.

6. The system of claim 4 or 5, wherein:
the inlet comprises a plurality of bent portions; and
the heating device surrounds the plurality of bent portions.

7. The system of anyone of claims 4-6, wherein:
the heating device comprises a coil-shaped heat radiating device; and
the inlet passes through a region surrounded by the coil-shaped heat radiating device.

8. The system of anyone of claims 3-7, wherein:
each of the inlet and the outlet comprises a blocking member; and
the controller is further configured to control a flow of the target gas through the inlet and the outlet by controlling each of the blocking members of the inlet and the outlet to be open or closed.

9. The system of anyone of claims 3-8, wherein:
each of the inlet and the outlet comprises a pump device; and
the controller is further configured to control the pump device of the inlet or the pump device of the outlet to regulate a speed and a flow rate of the target gas through the inlet and the outlet.

10. The system of anyone of claims 1-9, further comprising an air tank and a valve, wherein:
the controller is further configured to operate the valve to inject a preset gas contained in the air tank into the chamber; and
temperature and humidity of the preset gas are adjusted to predetermined values, wherein, optionally, the sensor is further configured to perform an initialization using the preset gas contained in the chamber.

11. The system of anyone of claims 1-10, further comprising an outside air intake and a valve, wherein the controller is further configured to operate the valve to inject outside air drawn through the outside air intake into the chamber, wherein, optionally, the sensor is further configured to perform an initialization using the outside air contained in the chamber.

12. The odor detection system of claim 1, wherein the sensor is further configured to detect the odor from a mixed gas of the target gas and remaining gas in the chamber in response to the remaining gas and the target gas being mixed within the chamber.

13. The system of claim 12,
wherein the sensor is further configured to start operations to detect the odor in response to a temperature and a humidity of the mixed gas reaching a target value,
and/or
wherein the sensor is further configured to start operations to detect the odor in response to a speed or a flow rate of the mixed gas becoming stable.

14. The odor detection system of claim 1, wherein the chamber assembly further comprises an inlet and an outlet, wherein the system further comprises a circulation flow path coupled with the inlet and the outlet, wherein the controller is configured to control the temperature and the humidity of the target gas and inflow the target gas into the chamber through the inlet, outflow the target gas from the chamber through the outlet, and control a pump member to circulate the target gas within a closed path defined by the circulation flow path, wherein, optionally, the sensor is further configured to detect the odor while the target gas is circulating within the closed path or to detect the odor after the target gas has circulated within the closed path for a predetermined time.

15. The system of claim 14, wherein:
the controller is further configured to block blocking members of the inlet and the outlet in response to the odor being detected by the sensor; and
the blocking members are disposed on one side of the inlet close to the chamber and on one side of the outlet close to the chamber.
